# EUROPEAN PATENT SPECIFICATION

(11) **EP 3 020 430 B1**
(45) Date of publication and mention of the grant of the patent: **28.02.2018**
(21) Application number: 15810725.0
(22) Date of filing: 04.06.2015
(51) Int. Cl.: A61B 17/34, A61B 17/06

(54) **CANNULA FOR CATGUT-EMBEDDING THERAPY**
KANÜLE FÜR CATGUT-EINBETTUNGSTHERAPIE
CANULE POUR THÉRAPIE D'INTÉGRATION DE CATGUT

(30) Priority: 24.06.2014 KR 20140004768 U
(43) Date of publication of application: 18.05.2016
(73) Proprietor: Hong, You Ree, Daegu 42074 (KR)
(72) Inventor: Hong, You Ree, Daegu 42074 (KR)
(74) Representative: Jackson, Robert Patrick
(86) International application number: PCT/KR2015/005629
(87) International publication number: WO 2015/199348

(56) References cited:
- JP-B2- 4 897 149
- KR-A- 20120 128 443
- KR-A- 20130 020 123
- KR-B1- 100 614 846
- KR-B1- 101 371 948

## Description

### [Technical Field]

The present invention relates to a cannula for thread embedding therapy, and, more particularly, to a cannula for thread embedding therapy which enables an embedding thread having thorns formed therein to easily pass through a hole.

### [Background Art]

Hereinafter, the background art of the present invention will be described with reference to the accompanying drawings.

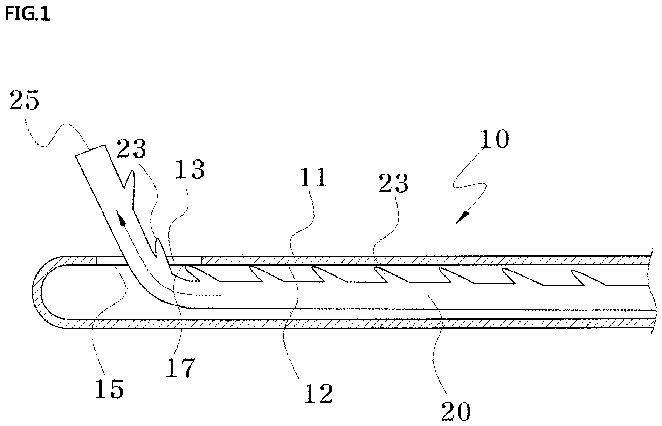

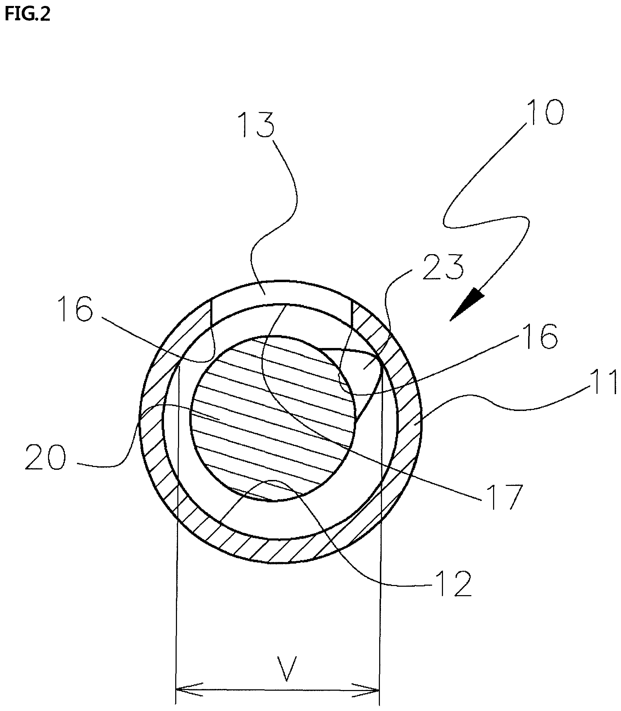

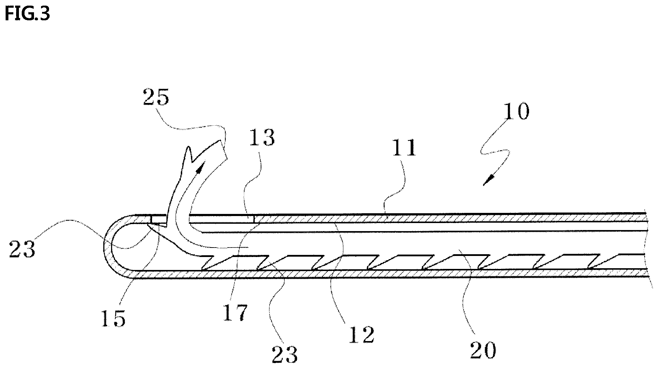

FIG. 1 is a cross-sectional view of the prior art showing a cutaway of a tube in a length direction in a process of passing an embedding thread through a hole of a cannula in a state in which thorns face upward, FIG. 2 is a cross-sectional view of the prior art showing a cutaway of a tube in a width direction in a process of passing an embedding thread through a hole of a cannula in a state in which thorns face a lateral side of the tube, and FIG. 3 is a cross-sectional view of the prior art showing a cutaway of a tube in a length direction while thorns are caught on a front end of a hole of a cannula in a process of passing an embedding thread through the hole in a state in which the thorns face downward.

In recent years, thread embedding therapy has come into the spotlight due to its ability to lift wrinkles using a simple surgical procedure of inserting an embedding thread 20 under the skin. Such a surgical procedure has been recognized as safe because the surgical procedure can be used to lift up wrinkles without incising the skin or subcutaneously injecting a toxic substance such as botulinum toxin.

This thread embedding therapy was first realized using injection needles having pointed heads, but recently cannulas 10 have been used. A cannula 10 includes a tube 11 having a blunt head, and a hole 13 formed at a lateral side of a leading end of the tube 11. Cannulas 10 were first used to stick needles into the skin and suck out body fluids or fat or to administer liquid medicine.

In thread embedding therapy, cannulas 10 have come to be preferred to injection needles because injection needles cause pain and may damage nerves when inserted subcutaneously due to their pointed heads, while cannulas 10 do not have such problems due to their blunt heads.

When such a cannula 10 is used for thread embedding therapy, an embedding thread 20 with thorns 23 is put into a hole 13 of the cannula 10 so that the embedding thread 20 is accommodated in the hole 13. Then, the embedding thread 20 is drawn out of the hole 13 in a state in which a portion of the embedding thread 20 is exposed to the outside.

By doing this, the drawn embedding thread 20 may be pressurized by pressing a leading end of the cannula 10 after the cannula 10 is inserted under the skin, and the embedding thread 20 may be allowed to reside in the hole 13 by drawing the cannula 10.

In the surgical procedure, thorns 23 are formed on the embedding thread 20 to face one lateral side of the tube 11. When the embedding thread 20 is inserted into the hole 13, the thorns 23 face forward. As a result, the thorns 23 of the embedding thread 20 drawn through the hole 13 face backward, which may reduce pain because the thorns 23 lie flat while the embedding thread 20 is passed through the skin upon subcutaneous insertion. In this case, no pain is caused because the embedding thread 20 held in the tube 11 does not come in contact with subcutaneous tissues even when the thorns 23 face forward. When the thorns 23 of the embedding thread 20 inserted into the tube 11 face backward, the thorns 23 drawn through the hole 13 face backward, thereby causing pain upon subcutaneous insertion.

Therefore, a surgical operator often performs a surgical procedure by further drawing the embedding thread 20 as necessary in a state in which the embedding thread 20 is inserted into the hole 13 of the cannula 10. In this case, since the embedding thread 20 is twisted in the tube 11, the thorns 23 often face upward or downward or face a lateral side of the tube 11. Since the embedding thread 20 is formed of a synthetic resin, is an elongated linear element, but is not formed straightly like a hard wire, the embedding thread 20 rotates and is twisted when inserted into the tube 11.

However, since the embedding thread 20 is bent by a rear end 17 of the hole 13 when the thorns 23 face upward, the embedding thread 20 may be easily drawn out.

Also, an inner lateral surface 12 of the tube 11 has a circular shape when the thorns 23 face a lateral side of the tube 11, as shown in FIG. 2. Therefore, since the width V of the inner lateral surface 12 of the tube 11 narrows when the inner lateral surface 12 of the tube 11 faces upward, the thorns 23 may be bent, and the embedding thread 20 may thereby be easily drawn.

However, the thorns 23 get caught on a front end 15 of the hole 13 when the thorns 23 face downward, as shown in FIG. 3, which makes it impossible to draw the embedding thread 20. KR20130020123 A also discloses a surgical instrument for use during face wrinkle plastic surgery in operation theatre, wherein the instrument has a surgical thread comprising a thread body and an injecting needle comprising a slender tube.

### [Disclosure]

### [Technical Problem]

Therefore, it is an aspect of the present invention to provide a cannula for thread embedding therapy capable of solving a problem in which it is difficult to draw an embedding thread having thorns formed therein because the thorns get caught on a front end of the hole when the embedding thread is re-drawn to an extent required for a surgical procedure in a state in which the embedding thread is inserted into a hole.

### [Technical Solution]

According to an aspect of the present invention, there is provided a cannula for thread embedding therapy, which includes a tube having a closed leading end, and a hole formed at a lateral side of the tube so that an embedding thread passes through the hole, wherein the cannula further includes a ball inserted into and fixed inside the tube and disposed to guide the embedding thread through the hole.

In this case, a rear end of the ball may be disposed to correspond to a front end of the hole.

Also, the ball may be disposed to overlap the front end of the hole.

In addition, the cannula may further include the embedding thread inserted through a rear portion of the tube and drawn through the hole.

Further, the embedding thread may have thorns 131 arranged at a side thereof in a length direction.

### [Advantageous Effects]

The cannula for thread embedding therapy according to the present invention has an effect of solving a problem in which it is impossible to draw an embedding thread having thorns formed therein because the thorns get caught on a front end of a hole formed at a front portion of a tube when the embedding thread is inserted into a rear portion of the tube and drawn through the hole.

### [Description of Drawings]

FIG. 1 is a cross-sectional view of the prior art showing a cutaway of a tube in a length direction in a process of passing an embedding thread through a hole of a cannula in a state in which thorns face upward.
FIG. 2 is a cross-sectional view of the prior art showing a cutaway of a tube in a width direction in a process of passing an embedding thread through a hole of a cannula in a state in which thorns face a lateral side of the tube.
FIG. 3 is a cross-sectional view of the prior art showing a cutaway of a tube in a length direction while thorns are caught on a front end of a hole of a cannula in a process of passing an embedding thread through the hole in a state in which the thorns face downward.
FIG. 4 is a cross-sectional view showing a cutaway of a tube in a length direction in a process of moving an embedding thread forward a ball inside the tube in a cannula for thread embedding therapy according to the present invention, in which (A) shows the ball closely attached to an inner leading end of the tube, and (B) shows the ball spaced apart from the inner leading end of the tube.
FIG. 5 is a cross-sectional view showing a cutaway of the tube in a length direction in a process of passing the embedding thread through a hole via the ball in a state in which thorns face downward in the cannula for thread embedding therapy according to the present invention.
FIG. 6 is a perspective view showing the embedding thread drawn through the hole in the cannula for thread embedding therapy according to the present invention.
FIG. 7 is a cross-sectional view showing a surgical procedure of maintaining the embedding thread under the skin using the cannula for thread embedding therapy according to the present invention.

### [Best Mode]

Hereinafter, embodiments and use examples of the present invention will be described in detail with reference to the accompanying drawings.

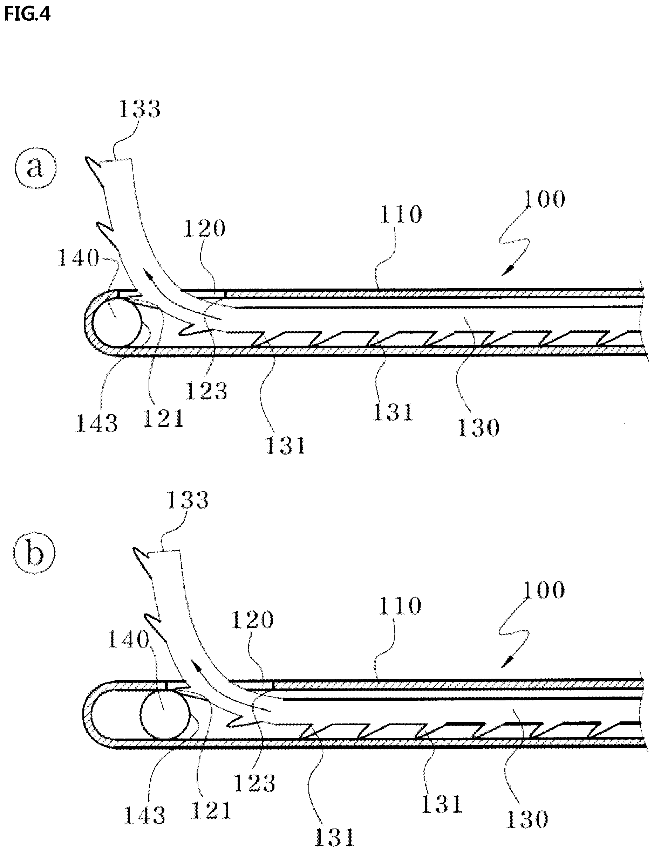

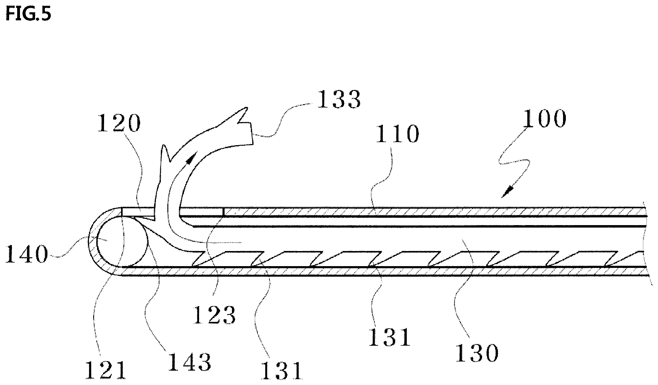

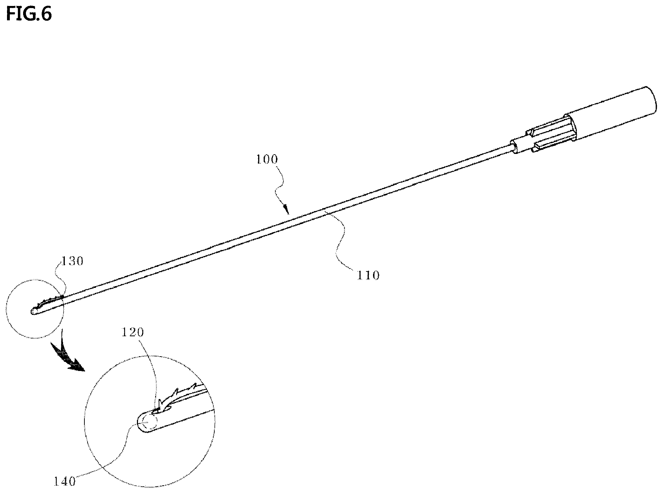

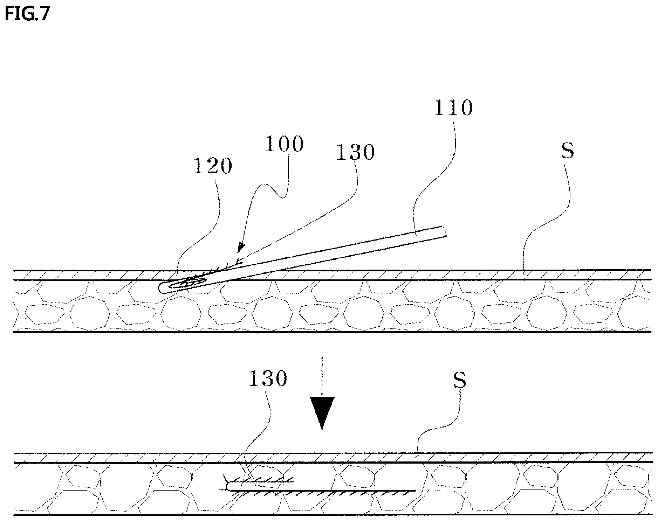

FIG. 4 is a cross-sectional view showing a cutaway of a tube in a length direction in a process of moving an embedding thread forward a ball inside the tube in a cannula for thread embedding therapy according to the present invention, in which (A) shows the ball closely attached to an inner leading end of the tube, and (B) shows the ball spaced apart from the inner leading end of the tube, FIG. 5 is a cross-sectional view showing a cutaway of the tube in a length direction in a process of passing the embedding thread through a hole via the ball in a state in which thorns face downward in the cannula for thread embedding therapy according to the present invention, FIG. 6 is a perspective view showing the embedding thread drawn through the hole in the cannula for thread embedding therapy according to the present invention, and FIG. 7 is a cross-sectional view showing a surgical procedure of maintaining the embedding thread under the skin using the cannula for thread embedding therapy according to the present invention.

In general, a cannula includes a tube 110 having a blunt head, and a hole 120 formed at a lateral side of the tube 110 for an embedding thread 130 to pass through. As an example, the tube 110 may be formed in a cylindrical shape.

The present invention is characterized in that the cannula is configured to prevent a situation in which thorns 131 get caught on the hole 120 when the embedding thread 130 is inserted into and drawn from the hole 120.

For this purpose, the cannula according to the present invention has the following configuration.

The cannula includes a ball 140 that is inserted into and fixed inside the tube 110 and disposed to guide the embedding thread 130 through the hole 120.

The ball 140 may be configured so that a rear end 143 of the ball 140 corresponds to a front end 121 of the hole 120 to guide the embedding thread 130 through the hole 120. Also, the ball 140 may be configured to overlap the front end 121 of the hole 120.

As an example, the ball 140 may be configured to be inserted into the tube 110 by press fitting, or may be fixed in the tube 110 by means of an adhesive. Also, the ball 140 may be configured to be pressed by pressurizing the tube 110.

Also, the cannula includes the embedding thread 130 which is inserted into and then drawn from the hole 120 of the tube 110.

The embedding thread 130 has thorns 131 arranged at a lateral side thereof in a length direction. As an example, the thorns 131 may be formed to be bent toward one lateral side of the tube 110. In this case, the thorns 131 face one lateral side of the tube 110 because a cutting blade is formed to cut into a body of the embedding thread 130.

A use example of the cannula having the configuration according to the present invention will be described as follows.

The embedding thread 130 is inserted into the hole 120 of the tube 110 to be accommodated behind the ball 140. Thereafter, a portion of the embedding thread 130 is exposed through the hole 120. Then, a surgical operator performs a surgical procedure by grabbing the exposed portion of the embedding thread 130 and drawing the embedding thread 130 to an extent that is necessary. In this case, the thorns 131 face upward, downward and toward left and right lateral sides of the tube 110 as the embedding thread 130 rotates in the tube 110.

When the embedding thread 130 is drawn out of the hole 120 in this way, the embedding thread 130 can be continuously drawn when the thorns 131 face upward because the thorns 131 press on the rear end 123 of the hole 120.

Also, when the thorns 131 face toward one lateral side of the tube 110, an inner lateral surface of the tube 110 is in a circular shape. Therefore, since the width of the inner lateral surface narrows when the inner lateral surface faces upward, the embedding thread 130 may be naturally bent, and easily drawn.

In addition, when the thorns 131 face downward as shown in FIG. 4, the thorns 131 are drawn upward while colliding with the round curved surface of the ball 140. Therefore, the embedding thread 130 may be easily drawn in a continuous manner.

The situation described above in which the thorns 131 get caught on the front end 121 of the hole 120 may be prevented when the embedding thread 130 is drawn out of the tube 110 to adjust a drawing length of the embedding thread 130 prior to a surgical procedure after the embedding thread 130 is inserted into the hole 120 of the tube 110.

One surgical procedure example of the cannula having the configuration according to the present invention will be described next with reference to FIG. 7.

In a state in which the embedding thread 130 is drawn out of the hole 120 of the tube 110, first, an injection needle is inserted into and removed from the skin. Thereafter, the tube 110 is inserted into a site of the skin into which the injection needle was inserted to push the embedding thread 130 into the skin. In this case, since the head of the tube 110 is blunt, pain may be relieved and nerve damage may be prevented compared to the injection needle. After the tube 110 is inserted to a proper depth in this way, the leading end of the tube 110 is pushed so that the embedding thread 130 is drawn out of the hole 120 and presses the skin S. Then, the tube 110 is removed. As a result, the embedding thread 130 may remain under the skin.

Since the thorns 131 inserted into the tube 110 in the surgical procedure face forward, the thorns 131 of the embedding thread 130 drawn out of the hole 120 face a rear portion of the tube 110. Therefore, when the tube 110 is inserted into the skin, the thorns 131 of the drawn embedding thread 130 lie flat, and the thorns 131 in the tube 110 may remain in an erect posture. Therefore, the surgical procedure may cause less pain to the patient. When the thorns 131 of the drawn embedding thread 130 face forward, the thorns 131 may be in an erect posture upon subcutaneous insertion, which may cause pain.

As described above, when the embedding thread 130 is pulled to erect the thorns 131 in a state in which one strand of the embedding thread 130 is drawn out of the skin S while the embedding thread 130 remains under the skin S, the thorns 131 that lie flat under the skin S may be erected so that the embedding thread 130 can be easily held under the skin S.

The embodiments and the accompanying drawings provided and shown herein are merely examples which exemplarily describe the scope of the present invention. Therefore, it is apparent that the scope of the present invention is not limited to the embodiments thereof since the embodiments disclosed herein are intended to describe the scope of the present invention, but not intended to limit the scope of the present invention. Accordingly, it will be apparent to those skilled in the art that various changes and modifications can be made to the above-described embodiments of the present invention without departing from the scope of the present invention. Thus, it should be understood that the present invention covers all such changes and modifications provided they come within the scope of the appended claims.

## Claims

1. A cannula for thread embedding therapy, comprising:
a tube (110) having a closed leading end; and
a hole (120) formed at a lateral side of the tube (110) for an embedding thread (130) to pass through, **characterised in that** the cannula further comprises a ball (140) inserted into and fixed inside the tube (110) and disposed to guide the embedding thread (130) through the hole (120).

2. The cannula according to claim 1, wherein the ball (140) is disposed to overlap a front end (121) of the hole (120).

3. The cannula according to claim 1, wherein a rear end (143) of the ball (140) is disposed to correspond to the front end (121) of the hole (120).

4. The cannula according to any one of claims 1 to 3, further comprising the embedding thread (130) drawn through the hole (120).

5. The cannula according to claim 4, wherein the embedding thread (130) has thorns (131) arranged at a side thereof in a length direction.

## Patentansprüche

1. Kanüle für die Fadeneinbettungstherapie, umfassend:
eine Röhre (110), die ein geschlossenes vorderes Ende aufweist; und
ein Loch (120), das an einer seitlichen Seite der Röhre (110) ausgebildet ist, durch das ein Einbettungsfaden (130) verlaufen kann,
**dadurch gekennzeichnet, dass**
die Kanüle weiter eine Kugel (140) umfasst, die in die Röhre (110) eingesetzt und dort befestigt ist und die angeordnet ist, den Einbettungsfaden (130) durch das Loch (120) zu führen.

2. Kanüle nach Anspruch 1, wobei die Kugel (140) so angeordnet ist, dass sie sich mit einem vorderen Ende (121) des Loches (120) überlappt.

3. Kanüle nach Anspruch 1, wobei ein hinteres Ende (143) der Kugel (140) so angeordnet ist, dass sie dem vorderen Ende (121) des Loches (120) entspricht.

4. Kanüle nach einem der Ansprüche 1 bis 3, weiterhin umfassend den durch das Loch (120) gezogenen Einbettungsfaden (130).

5. Kanüle nach Anspruch 4, wobei der Einbettungsfaden (130) Dornen (131) aufweist, die auf einer seiner Seiten in Längenrichtung angeordnet sind.

## Revendications

1. Canule pour thérapie d'intégration de fil, comprenant :
un tube (110) ayant une extrémité avant fermée ; et
un trou (120) formé au niveau d'un côté latéral du tube (110) prévu pour le passage à travers celui-ci d'un fil à intégrer (130),
**caractérisée en ce que**
la canule comprend en outre une bille (140) insérée dans et fixée à l'intérieur du tube (110) et disposée pour guider le fil à intégrer (130) à travers le trou (120).

2. Canule selon la revendication 1, dans laquelle la bille (140) est disposée pour chevaucher une extrémité avant (121) du trou (120).

3. Canule selon la revendication 1, dans laquelle une extrémité arrière (143) de la bille (140) est disposée pour correspondre à l'extrémité avant (121) du trou (120).

4. Canule selon l'une quelconque des revendications 1 à 3, comprenant en outre le fil à intégrer (130) passant à travers le trou (120).

5. Canule selon la revendication 4, dans lequel le fil à intégrer (130) a des pointes (131) agencées au niveau d'un côté de celui-ci dans une direction de longueur.
